# EUROPÄISCHE PATENTSCHRIFT

(11) **EP 1 722 754 B1**
(45) Veröffentlichungstag und Bekanntmachung des Hinweises auf die Patenterteilung: **15.08.2007**
(21) Anmeldenummer: 04797929.9
(22) Anmeldetag: 16.11.2004
(51) Int. Cl.: A61K 8/49, A61Q 5/08, A61Q 5/10, C09B 67/00

(54) **HAARFÄRBEMITTEL ZUM GLEICHZEITIGEN FÄRBEN UND AUFHELLEN VON KERATINFASERN**
HAIR DYE FOR DYEING AND LIGHTENING KERATIN FIBERS
AGENTS COLORANTS CAPILLAIRES PERMETTANT A LA FOIS DE COLORER ET D'ECLAIRCIR DES FIBRES DE KERATINE

(30) Priorität: 21.02.2004 DE 102004008604
(43) Veröffentlichungstag der Anmeldung: 22.11.2006
(73) Patentinhaber: Wella Aktiengesellschaft, 64274 Darmstadt (DE)
(72) Erfinder: PASQUIER, Cécile, CH-1723 Marly (CH); KIENER, Caroline, CH-1723 Marly (CH); BRAUN, Hans-Jürgen, CH-3182 Ueberstorf (CH)
(74) Vertreter: Bockhorst, Matthias
(86) Internationale Anmeldenummer: PCT/EP2004/012982
(87) Internationale Veröffentlichungsnummer: WO 2005/079732

(56) Entgegenhaltungen:
- DE-A1- 2 822 912
- DE-A1- 3 602 587
- DE-A1- 10 118 271

## Beschreibung

Gegenstand der vorliegenden Erfindung sind Mittel zum gleichzeitigen Färben und Aufhellen von Keratinfasern, wie zum Beispiel Wolle, Seide oder Haaren und insbesondere menschlichen Haaren, welche mindenstens einen Thiazoliumazofarbstoff enthalten.

Für die farbverändernde Behandlung von Keratinfasern werden in der Regel zwei Färbeverfahren angewendet. Im ersten Verfahren wird die Färbung mit sogenannten oxidativen oder permanenten Färbemitteln unter Verwendung einer Mischung aus verschiedenen Entwicklersubstanzen und Kupplersubstanzen und eines Oxidationsmittels erzeugt. Bei Bedarf können bei diesem Verfahren zur Abrundung des Färbeergebnisses oder zur Erzeugung von besonderen Farbeffekten sogenannte direktziehende (nicht-oxidative) Farbstoffe zugesetzt werden. Das zweite Verfahren bedient sich ausschließlich direktziehender Farbstoffe, die in einer geeigneten Trägermasse auf die Fasern aufgebracht werden. Dieses Verfahren ist einfach anzuwenden, ausgesprochen schonend und zeichnet sich durch eine geringe Schädigung der Keratinfaser aus. An die hierbei verwendeten direktziehenden Farbstoffe werden eine Vielzahl von Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Färbungen eine gute Lichtechtheit, Säureechtheit und Reibechtheit gefordert.

Im Vergleich zu oxidativen Färbungen besitzen nicht-oxidative Färbungen in der Regel jedoch eine geringere Haltbarkeit und einen schlechteren Farbausgleich. Zudem sind direktziehende Färbemittel in der Regel nicht in der Lage das Haar "hellerzufärben", da viele Direktzieher die zum Aufhellen benötigten Oxidationsmittel und/oder den erforderlichen pH-Wert von größer/gleich 9 nicht vertragen.

DE 101 18 271 A1 offenbart Haanfärbemittel enthaltend einen Thiazoliumazofarbstoff, der sich jedoch von den Verbindungen der vorliegenden Anmeldung strukturell unterscheidet.

Aufgabe der vorliegenden Erfindung ist es daher, ein Färbemittel auf der Basis von gegenüber basischen pH-Werten und Oxidationsmitteln beständigen direktziehenden Farbstoffen -insbesondere für den blauen Farbbereich- zur Verfügung zu stellen.

Es wurde nunmehr gefunden, dass diese Aufgabe durch den Einsatz von bestimmten Thiazoliumazofarbstoffen gelöst werden kann.

Gegenstand der vorliegenden Erfindung ist daher ein Mittel zur gleichzeitigen Aufhellung und Färbung von Keratinfasern -insbesondere Haaren-, welches dadurch gekennzeichnet ist, dass es (a) ein Oxidationsmittel sowie (b) mindestens einen Thiazoliumazofarbstoff der Formel (I) enthält, und (c) einen basischen pH-Wert aufweist, 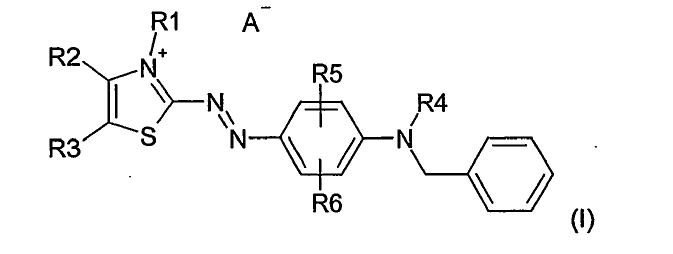 wobei
R1 eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt;
R2 und R3 gleich oder verschieden sein können und unabhängig voneinander Wasserstoff, ein Halogenatom (F, Cl, Br, J), eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe, oder eine substituierte oder unsubstituierte Heteroarylgruppe darstellen;
R4 gleich Wasserstoff, einer gesättigten oder ungesättigten (C₁-C₁₂)-Alkylgruppe, einer mit einem Halogenatom (F, Cl, Br, J) substituierten (C₁-C₁₂)-Alkylgruppe, einer Hydroxy-(C₁-C₁₂)-alkylgruppe, einer Amino-(C₁-C₁₂)-alkylgruppe oder einer Benzylgruppe ist;
R5 und R6 gleich oder verschieden sein können und unabhängig voneinander Wasserstoff, ein Halogenatom (F, Cl, Br, J), eine gesättigte oder ungesättigte (C₁-C₁₂)-Alkylgruppe, eine Hydroxygruppe, eine (C₁-C₁₂)-Alkoxygruppe, eine Cyanogruppe, eine Nitrogruppe, eine Aminogruppe, eine (C₁-C₁₂)-Alkylaminogruppe, oder eine Di(C₁-C₁₂)-alkylaminogruppe darstellen; und
A⁻ gleich einem Anion einer organischen oder anorganischen Säure ist.

Unter den vorgenannten Verbindungen der Formel (I) sind solche bevorzugt, bei denen R1 gleich einer gesättigten oder ungesättigten (C₁-C₁₂)-Alkylgruppe ist, wobei Verbindungen der Formel (I), bei denen R1 gleich einer gesättigten oder ungesättigten (C₁-C₁₂)-Alkylgruppe und R4 gleich einer gesättigten oder ungesättigten (C₁-C₁₂)-Alkylgruppe ist, besonders bevorzugt sind.
A⁻ ist vorzugsweise gleich Chlorid, Bromid, Jodid, Hydrogensulfat, Sulfat, Toluolsulfonat, Benzolsulfonat, Monomethylsulfat, Hexafluorphosphat, Hexafluorantimonat, Tetrafluorborat, Tetraphenylborat, Formiat, Acetat oder Propionat, wobei das Chloridion, das Bromidion, das Monomethylsulfation und das Acetation besonders bevorzugt sind.

Als geeignete Verbindung der allgemeinen Formel (I) können beispielsweise genannt werden:
3-Methyl-2-[[4-[methyl(phenylmethyl)amino]phenyl]azo]-thiazoliumchlorid, 3-Methyl-2-[[4-[methyl(phenylmethyl)amino]phenyl]azo]-thiazolium-bromid, 3-Methyl-2-[[4-[methyl(phenylmethyl)amino]-phenyl]azo]-thiazolium-monomethylsulfat, 3-Methyl-2-[[4-[methyl- . (phenylmethyl)amino]phenyl]-azo]-thiazolium-acetat, 3,4-Dimethyl-2-[[4-[methyl(phenylmethyl)amino]phenyl]azo]-thiazolium-chlorid, 3,4-Dimethyl-2-[[4-[methyl(phenylmethyl)amino]phenyl]azo]-thiazolium-bromid, 3,4-Dimethyl-2-[[4-[methyl(phenylmethyl)amino]phenyl]azo]-thiazolium-monomethylsulfat, 3,4-Dimethyl-2-[[4-[methyl(phenylmethyl)-amino]phenyl]azo]-thiazolium-acetat, 3,5-Dimethyl-2-[[4-[methyl-(phenylmethyl)-amino]phenyl]azo]-thiazolium-chlorid, 3,5-Dimethyl-2-[[4-[methyl-(phenylmethyl)amino]phenyl]azo]-thiazolium-bromid, 3,5-Dimethyl-2-[[4-[methyl(phenylmethyl)amino]phenyl]azo]-thiazolium-monomethylsulfat, 3,5-Dimethyl-2-[[4-[methyl(phenylmethyl)amino]-phenyl]azo]-thiazolium-acetat, 3,4,5-Trimethyl-2-[[4-[methyl-(phenyl-methyl)-amino]phenyl]azo]-thiazolium-chlorid, 3,4,5-Trimethyl-2-[[4-[methyl-(phenylmethyl)amino]phenyl]azo]-thiazolium-bromid, 3,4,5-Trimethyl-2-[[4-[methyl(phenylmethyl)amino]phenyl]azo]-thiazolium-monomethylsulfat, 3,4,5-Trimethyl-2-[[4-[methyl(phenylmethyl)amino]-phenyl]azo]-thiazolium-acetat, 2-[[4-[Ethyl-(phenylmethyl)amino]2-methylphenyl]azo]-3-methyl-thiazolium-chlorid, 2-[[4-[Ethyl(phenylmethyl)-amino]2-methylphenyl]azo]-3-methyl-thiazolium-bromid, 2-[[4-[Ethyl-(phenylmethyl)amino]2-methylphenyl]azo]-3-methyl-thiazolium-monomethylsulfat, 2-[[4-[Ethyl(phenyl-methyl)amino]2-methylphenyl]azo]-3-methyl-thiazolium-acetat, 2-[[4-[Ethyl(phenylmethyl)-amino]2-methylphenyl]azo]-3,4-dimethyl-thiazolium-chlorid, 2-[[4-[Ethyl-(phenylmethyl)-amino]2-methylphenyl]azo]-3,4-dimethyl-thiazolium-bromid, 2-[[4-[Ethyl(phenylmethyl)-amino]2-methylphenyl]azo]-3,4-dimethyl-thiazolium-monomethylsulfat, 2-[[4-[Ethyl(phenylmethyl)-amino]2-methylphenyl]azo]-3,4-dimethyl-thiazolium-acetat, 2-[[4-[Ethyl(phenylmethyl)-amino]2-methylphenyl]azo]-3,5-dimethyl-thiazolium-chlorid, 2-[[4-[Ethyl(phenylmethyl)-amino]2-methylphenyl]azo]-3,5-dimethyl-thiazolium-bromid, 2-[[4-[Ethyl(phenylmethyl)-amino]2-methylphenyl]azo]-3,5-dimethyl-thiazolium-monomethylsulfat, 2-[[4-[Ethyl(phenylmethyl)-amino]2-methylphenyl]azo]-3,5-dimethyl-thiazolium-acetat, 2-[[4-[Ethyl(phenylmethyl)amino]2-methyl-phenyl]azo]-3,4,5-trimethyl-thiazolium-chlorid, 2-[[4-[Ethyl(phenylmethyl)-amino]2-methylphenyl]azo]-3,4,5-trimethyl-thiazolium-bromid, 2-[[4-[Ethyl-(phenylmethyl)amino]2-methylphenyl]azo]-3,4,5-trimethyl-thiazolium-monomethylsulfat, 2-[[4-[Ethyl-(phenylmethyl)amino]2-methylphenyl]azo]-3,4,5-trimethyl-thiazolium-acetat, 3-Methyl-2-[[4-[(phenylmethyl)amino]-phenyl]azo]-thiazolium-chlorid, 3-Methyl-2-[[4-[(phenyl-methyl)amino]-phenyl]azo]-thiazolium-bromid, 3-Methyl-2-[[4-[(phenyl-methyl)amino]-phenyl]azo]-thiazolium-monomethylsulfat, 3-Methyl-2-[[4-[(phenylmethyl)amino]phenyl]azo]-thiazolium-acetat, 3,4-Dimethyl-2-[[4-[(phenylmethyl)amino]phenyl]azo]-thiazolium-chlorid, 3,4-Dimethyl-2-[[4-[(phenylmethyl)amino]phenyl]azo]-thiazolium-bromid, 3,4-Dimethyl-2-[[4-[(phenylmethyl)amino]phenyl]azo]-thiazolium-monomethylsulfat, 3,4-Dimethyl-2-[[4-[(phenylmethyl)amino]phenyl]azo]-thiazolium-acetat, 3,5-Dimethyl-2-[[4-[(phenylmethyl)amino]phenyl]azo]-thiazolium-chlorid, 3,5-Dimethyl-2-[[4-[(phenylmethyl)amino]phenyl]azo]-thiazolium-bromid, 3,5-Dimethyl-2-[[4-[(phenylmethyl)amino]phenyl]azo]-thiazolium-monomethylsulfat, 3,5-Dimethyl-2-[[4-[(phenylmethyl)amino]phenyl]azo]-thiazolium-acetat, 3,4,5-Trimethyl-2-[[4-[(phenylmethyl)amino]phenyl]azo]-thiazoliumchlorid, 3,4,5-Trimethyl-2-[[4-[(phenylmethyl)amino]phenyl]azo]-thiazoliumbromid, 3,4,5-Trimethyl-2-[[4-[(phenylmethyl)amino]phenyl]azo]-thiazolium-monomethylsulfat, 3,4,5-Trimethyl-2-[[4-[(phenylmethyl)amino]phenyl]azo]-thiazoliumacetat, 2-[[4-[Bis(phenylmethyl)amino]phenyl]azo]-3-methylthiazoliumchlorid, 2-[[4-[Bis(phenylmethyl)amino]phenyl]azo]-3-methylthiazolium-bromid, 2-[[4-[Bis(phenylmethyl)amino]phenyl]azo]-3-methylthiazolium-monomethylsulfat, 2-[[4-[Bis(phenylmethyl)amino]phenyl]azo]-3-methyl-thiazolium-acetat, 2-[[4-[Bis(phenylmethyl)amino]phenyl]azo]-3,4-dimethyl-thiazolium-chlorid, 2-[[4-[Bis(phenylmethyl)amino]phenyl]-azo]-3,4-dimethyl-thiazofium-bromid, 2-[[4-[Bis(phenylmethyl)amino]-phenyl]azo]- 3,4-dimethyl-thiazolium-monomethylsulfat, 2-[[4-[Bis(phenyl-methyl)amino]phenyl]azo]-3,4-dimethyl-thiazolium-acetat, 2-[[4-[Bis-(phenylmethyl)-amino]phenyl]azo]-3,5-dimethyl-thiazolium-chlorid, 2-[[4-[Bis(phenyl-methyl)amino]phenyl]azo]- 3,5-dimethyl-thiazolium-bromid, 2-[[4-[Bis-(phenylmethyl)amino]phenyl]azo]- 3,5-dimethylthiazolium-monomethylsulfat, 2-[[4-[Bis-(phenylmethyl)amino]phenyl]azo]-3,5-dimethyl-thiazolium-acetat, 2-[[4-[Bis(phenylmethyl)amino]phenyl]-azo]-3,4,5-trimethyl-thiazolium-chlorid, 2-[[4-[Bis(phenylmethyl)amino]-phenyl]azo]- 3,4,5-trimethyl-thiazolium-bromid, 2-[[4-[Bis(phenylmethyl)-amino]phenyl]azo]-3,4,5-trimethyl-thiazolium-monomethylsulfat und 2-[[4-[Bis(phenylmethyl)amino]phenyl]azo]-3,4,5-trimethyl-thiazolium-acetat.

Besonders bevorzugte Verbindungen der Formel (I) sind 3-Methyl-2-[[4-[methyl(phenylmethyl)amino]phenyl]azo]-thiazoliumchlorid, 3-Methyl-2-[[4-[methyl(phenylmethyl)amino]phenyl]azo]-thiazolium-bromid, 3-Methyl-2-[[4-[methyl(phenylmethyl)amino]phenyl]-azo]-thiazolium-monomethylsulfat, 3-Methyl-2-[[4-[methyl(phenylmethyl)-amino]phenyl]-azo]-thiazolium-acetat, 3,4-Dimethyl-2-[[4-[methyl(phenylmethyl)amino]phenyl]azo]-thiazolium-chlorid, 3,4-Dimethyl-2-[[4-[methyl(phenylmethyl)amino]phenyl]azo]-thiazolium-bromid, 3,4-Dimethyl-2-[[4-[methyl(phenylmethyl)amino]phenyl]azo]-thiazolium-monomethylsulfat, 3,4-Dimethyl-2-[[4-[methyl(phenylmethyl)-amino]phenil]azo]-thiazolium-acetat, 3,5-Dimethyl-2-[[4-[methyl(phenylmethyl)amino]-phenyl]azo]-thiazolium-chlorid, 3,5-Dimethyl-2-[[4-[methyl(phenylmethyl)-amino]-phenyl]azo]-thiazolium-bromid, 3,5-Dimethyl-2-[[4-[methyl(phenylmethyl)-amino]phenyl]azo]-thiazolium-monomethylsulfat, 3,5-Dimethyl-2-[[4-[methyl(phenylmethyl)amino]phenyl]azo]-thiazolium-acetat, 3,4,5-Trimethyl-2-[[4-[methyl-(phenylmethyl)-amino]phenyl]azo]-thiazolium-chlorid, 3,4,5-Trimethyl-2-[[4-[methyl-(phenylmethyl)amino]-phenyl]azo]-thiazolium-bromid, 3,4,5-Trimethyl-2-[[4-[methyl(phenylmethyl)amino]phenyl]azo]-thiazolium-monomethylsulfat, 3,4,5-Trimethyl-2-[[4-[methyl(phenylmethyl)amino]phenyl]azo]-thiazolium-acetat, 2-[[4-[Ethyl(phenylmethyl)amino]2-methyl-phenyl]azo]-3-methylthiazolium-chlorid, 2-[[4-[Ethyl(phenylmethyl)amino]-2-methylphenyl]azo]-3-methyl-thiazolium-bromid, 2-[[4-[Ethyl(phenyl-methyl)amino]-2methylphenyl]azo]-3-methyl-thiazolium-monomethylsulfat, 2-[[4-[Ethyl(phenyl-methyl)amino]2-methylphenyl]azo]-3-methylthiazolium-acetat, 2-[[4-[Ethyl(phenylmethyl)-amino]2-methylphenyl]azo]-3,4-dimethyl-thiazolium-chlorid, 2-[[4-[Ethyl(phenylmethyl)-amino]-2-methylphenyl]azo]-3,4-dimethyl-thiazolium-bromid, 2-[[4-[Ethyl(phenylmethyl)-amino]2-methylphenyl]azo]-3,4-dimethyl-thiazolium-monomethylsulfat, 2-[[4-[Ethyl(phenylmethyl)-amino]2-methylphenyl]azo]-3,4-dimethyf-thiazolium-acetat, 2-[[4-[Ethyl(phenylmethyl)-amino]2-methylphenyl]azo]-3,5-dimethyl-thiazolium-chlorid, 2-[[4-[Ethyl(phenylmethyl)-amino]2-methylphenyl]azo]-3,5-dimethyl-thiazolium-bromid, 2-[[4-[Ethyl(phenylmethyl)-amino]2-methylphenyl]azo]-3,5-dimethylthiazolium-monomethylsulfat, 2-[[4-[Ethyl(phenylmethyl)-amino]2-methylphenyl]azo]-3,5-dimethyl-thiazolium-acetat, 2-[[4-[Ethyl(phenylmethyl)amino]2-methylphenyl]azo]-3,4,5-trimethyl-thiazolium-chlorid, 2-[[4-[Ethyl(phenylmethyl)amino]2-methylphenyl]azo]-3,4,5-trimethylthiazolium-bromid und 2-[[4-[Ethyl(phenylmethyl)amino]2-methylphenyl]-azo]-3,4,5-trimethyl-thiazolium-monomethylsulfat und 2-[[4-[Ethyl-(phenylmethyl)amino]2-methylphenyl]azo]-3,4,5-trimethyl-thiazoliumacetat.

Die Verbindungen der Formel (I) sind in dem erfindungsgemäßen Färbemittel vorzugsweise in einer Menge von 0,01 bis 10 Gewichtsprozent, insbesondere 0,1 bis 8 Gewichtsprozent, enthalten.

Das erfindungsgemäße Färbemittel kann neben den Farbstoffen der Formel (I) zusätzlich noch weitere bekannte, gegenüber Oxidationsmitteln stabile, direktfärbende Farbstoffe enthalten, wie zum Beispiel 3-(2',6'-Diaminopyridyl-3'-azo)-pyridin (= 2,6-Diamino-3-((pyridin-3-yl)azo)pyridin), N,N-Di(2-hydroxyethyl)-3-methyl-4-((4-nitrophenyl)-azo)-anilin (Disperse Red 17, Cl 11210), 3-Diethylamino-7-(4-dimethylaminophenylazo)-5-phenyl-phenaziniumchlorid (Cl 11050), 4-(2-Thiazolylazo)-resorcin, 4-((4-Phenylamino)azo)benzosulfonsäurenatriumsalz (Orange IV), 1-((3-Aminopropyl)amino)-9,10-anthracendion (HC Red No. 8), 3',3",4,5,5',5",6,7-Octabromphenolsulfonphtalein (Tetrabromphenol Blue), 1-((4-Amino-3,5-dimethylphenyl)-(2,6-dichlorphenyl)-methylen)-3,5-dimethyl-4-imino-2,5-cyclo-hexadien-Phosphorsäure (1:1) (Basic Blue 77), 3',3",5',5"-Tetrabrom-m-kresolsulfonphthalein, 2,4-Dinitro-1-naphthol-7-sulfonsäure-Dinatriumsalz (Acid Yellow 1, Cl 10316), 4-[2'-Hydroxy-1'-naphthyl)azo]-benzosulfonsäure-Natriumsalz (Acid Orange 7, Cl 15510), 3',6'-Dihydroxy-2',4',5',7'-tetraiodospiro-[isobenzo-furan-1(3H), 9'(9H)-xanthen]-3-on-Dinatriumsalz (Acid Red 51, Cl 45430), 6-Hydroxy-5-((2-methoxy-5-methyl-4-sulfophenyl)azo)-2-Naphthalinsulfonsäure-dinatriumsalz (FD&C Red 40, Cl 16035), 2,4-Dinitro-1-naphthol-Natriumsalz (Acid Yellow 24; CI 10315), 2',4',5',7'-tetrabrom-4,5,6,7-tetrachlor-3',6'-dihydroxy-Spiro(isobenzofuran-1 (3H), 9'[9H]xanthen]-3-on-dinatriumsalz (Acid Red 92; Cl 45410), 4-(2-Hydroxy-1-naphthylazo)-3-methyl-benzolsulfonsäure-natriumsalz (Acid Orange 8, CI 15575), 2-Amino-1,4-naphthalindion, Dithizon (1,5-Diphenylthiocarbazon), N-(2-Hydroxyethyl))-2-nitro-4-trifluormethyl)anilin (HC Yellow 13), N-(2-hydroxyethyl)-4-nitro-anilin und 4-Chlor-N-(2,3-dihydroxypropyl)-2-nitro-anilin, 1-Methyl-4-((methylphenylhydrazono)methyl)pyridiniummethylsulfat (Basic Yellow No. 87), 3-((4,5-Dihydro-3-methyl-5-oxo-1-phenyl-1 H-pyrazol-4-yl)azo)-N,N,N-trimethyl-benzenaminium-chlorid, 3-[(3-Methyl-5-hydroxy-1-phenyl-1 H-pyrazol-4-yl)azo]-trimethylammonio-benzolchlorid (Basic Yellow No. 57), 2-((4-Aminophenyl)azo)-1,3-dimethyl-1 H-imidazol-3-ium-chlorid (Basic Orange No. 31), 1,4-Dimethyl-5-[(4-(dimethylamino)phenyl)azo]-1,2,4-triazolium-chlorid (Basic Red No. 22, CI11055), 2-((4-(Dimethylamino)phenyl)azo)-1,3-dimethyl-1 H-imidazoliumchlorid (Basic Red No. 51), 1,4-Dimethyl-5-[[4-[methyl(phenylmethyl)-amino]phenyl]azo]-1,2,4-triazolium-bromid (Basic Red No. 46), N,N,N-Trimethyl-3-{[4-(methylamino)-9,10-dioxo-9,10-dihydro-1-anthracenyl]-amino}-1-propanaminium-methylsulfat, N,N-Dimethyl-3-{[4-(methylamino)-9,10-dioxo-9,10-dihydro-1-anthracenyl]amino}-N-propyl-1-propanaminiumchlorid und N,N-Dimethyl-3-{[4-(methylamino)-9,10-dioxo-9,10-dihydro-1anthracenyl]amino}-N-propyl-1-propanaminium-bromid.

Der Gesamtgehalt an Farbstoffen der Formel (I) und direktfärbende Farbstoffen in dem erfindungsgemäßen Färbemittel beträgt etwa 0,01 bis 15 Gewichtsprozent, insbesondere etwa 0,1 bis 12 Gewichtsprozent.

Selbstverständlich können dem erfindungsgemäßen Färbemittel auch Oxidationsfarbstoffvorstufen, wie zum Beispiel o,p,m-Phenylendiamine, o,p,m-Aminophenole, Diphenole oder 4,5-Diaminopyrazole zugesetzt werden.

Die zusätzlichen Entwicklersubstanzen und Kupplersubstanzen können in dem Färbemittel jeweils in einer Gesamtmenge von etwa 0,01 bis 20 Gewichtsprozent, vorzugsweise etwa 0,1 bis 10 Gewichtsprozent und insbesondere 0,1 1 bis 5 Gewichtsprozent, enthalten sein.

Die Zubereitungsform des erfindungsgemäßen Färbemittels kann beispielsweise eine Lösung, insbesondere eine wässrige oder wässrigalkoholische Lösung sein, eine Creme, ein Gel, eine tensidhaltige schäumende Lösung (Shampoo, Aerosol), eine Emulsion oder ein anderer für die Anwendung auf dem Haar geeigneter, wasserhaltiger Träger sein. Es ist ebenfalls möglich, dass das erfindungsgemäße Färbemittel in Form von Pellets, Granulaten oder Pulvern vorliegt, die vor der Anwendung in einer wässrigen Zubereitung -beispielsweise in Wasser oder einer wässrigen Oxidationsmittelzubereitung- gelöst werden.

Die Zusammensetzung dieser Mittel stellt eine Mischung der Farbstoffkomponente mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielweise Ethylenglykol, Propylenglykol, 1,3-Propandiol, 1,4-Butandiol, 1,5-Pentandiol, 1,6-Hexandiol, 1,2,6-Hexantriol, Glycerin, Diethylenglykol, Dipropylenglykol, Polyalkylenglykole, wie Triethylenglykol, Polyethylenglykol, Tripropylenglykol, Polypropylenglykol, niedere Alkylether von mehrwertigen Alkoholen, wie Ethylenglykolmonomethylether, Ethylenglykolmonoethylether, Ethylenglykolmonopropylether, Ethylenglykolmonobuthylether, Diethylenglykolmonomethylether, Diethylenglykolmonoethylether, Triethylenglykolmonomethylether oder Triethylenglykolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon, und Diacetonalkohol, Ether, wie zum Beispiel Dibuthylether, Tetrahydrofuran, Dioxan, Diisopropylether, Ester wie zum Beispiel Ethylformiat, Methylformiat, Methylacetat, Ethylacetat, Propylacetat, Butylacetat, Phenylacetat, Ethylenglykolmonoethyletheracetat, und Essigsäurehydroxyethylester, Amide wie zum Beispiel Dimethylformamid und Dimethylacetamid, N-Methylpyrrolidon, sowie Harnstoff, Tetramethylharnstoff und Thiodiglykol.

Weiterhin können in dem erfindungsgemässen Färbemittel Netzmittel oder Emulgatoren aus den Klassen der anionischen, kationischen, amphoteren, nichtionogenen oder zwitterionischen oberflächenaktiven Substanzen wie Fettalkoholsulfate, oxethylierte Fettalkoholsulfate, Alkylsulfonate, Alkylbenzolsulfonate, α-Olefinsulfonate, Alkyltrimethylammoniumsalze, Alkylbetaine, oxethylierte Fettalkohole, oxethylierte Nonylphenole, Fettsäurealkanolamide, oxethylierte Fettsäureester, Fettalkoholpolyglykolethersulfate, Alkylpolyglucoside, Verdickungsmittel wie höhere Fettalkohole, Stärke, Cellulosederivate, Vaseline, Paraffinöl, Fettsäuren, und andere Fettkomponenten in Emulgieter Form, wasserlöslische polymere Verdickungsmittel wie natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie zum Beispiel Polyvinylalkohol, sowie außerdem Pflegestoffe wie Lanolinderivate, Cholesterin, Pantothensäure, wasserlöslische kationische Polymere, Proteinderivate, Provitamine, Vitamine, Pflanzenextrakte, Zucker und Betain, Hilfstoffe wie Feuchthaltmittel, Elektrolyte, Antioxidantien, Fettamide, Sequestrierungsmittel, filmbildende Agentien und Konservierungsmittel, enthalten sein. Neben Wasser kann auch ein wasserlöslisches organisches Lösungsmittel oder ein Gemisch derartiger Lösungsmittel sowie ein Wasser/Lösungsmittel-Gemisch verwendet werden.
Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Das gebrauchsfertige erfindungsgemäße Färbemittel wird unmittelbar vor Gebrauch durch Mischen der die Farbstoffe enthaltenden Farbträgermasse mit einem Oxidationsmittel hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12-prozentigen, vorzugsweise einer 3- bis 9-prozentigen, wässrigen Lösung, in Betracht. Das Gewichts-verhältnis zwischen Farbträgermasse und Oxidationsmittel beträgt hierbei vorzugsweise etwa 5:1 bis 1:3, insbesondere 1:1 bis 1:2. Größere Mengen an Oxidationsmittel werden vor allem bei höheren Konzentrationen an oxidativen Farbstoffvorstufen im Färbemittel, oder wenn gleichzeitig eine stärkere Bleichung der Keratinfaser (insbesondere der Haare) beabsichtigt ist, verwendet. Für den Fall, dass eine Aufhellung der Keratinfasern von bis zu 6 Tons tufen beabsichtigt ist, ist der Zusatz von Persulfaten, wie zum Beispiel Ammoniumpersulfat, Kaliumpersulfat oder Natriumpersulfat und deren Mischungen, möglich, sofern die verwendeten Farbstoffe der Formel (I) gegenüber Persulfaten stabil sind.

Das pH-Wert des gebrauchsfertigen erfindungsgemäßen Färbemittel stellt sich bei der Mischung der Farbträgermasse mit dem Oxidationsmittel auf einen pH-Wert ein, der durch die pH-Werte der Farbträgermasse des Oxidationsmittel sowie durch das Mischungsverhältnis bestimmt wird.
Das gebrauchsfertige Mittel weist einen basischen pH-Wert von größer 7, vorzugsweise einen pH-Wert von 8 bis 11, auf. Die basische Einstellung erfolgt hierbei vorzugsweise mit Ammoniak, wobei jedoch auch organische Amine, zum Beispiel 2-Amino-2-methyl-1-propanol, Tris(hydroxymethyl)aminomethan, Monoethanolamin und Triethanolamin, oder Mischungen von organischen Aminen und Ammoniak sowie anorganische Basen wie Natriumhydroxid, Kaliumhydroxid, Natriumcarbonat, Natriumhydrogencarbonat, Kaliumcarbonat, Kaliumhydrogencarbonat, Natriumphosphat, Borax (Na₂B₄O₇ x 10H₂O), Dinatriumhydrogenphosphat Verwendung finden können. Bei zu hohen pH-Werten kann mit anorganischen oder organischen Säuren, zum Beispiel Phosphorsäure, Essigsäure, Milchsäure, Ascorbinsäure, Zitronensäure oder Weinsäure, korrigiert werden.

Anschließend trägt man eine für die Färbebehandlung ausreichende Menge, im allgemeinen etwa 30 bis 120 Gramm, dieses Gemisches auf die Keratinfaser auf und läßt das Gemisch bei etwa 15 bis 50°C, vorzugsweise 30 bis 40 °C, etwa 1 bis 60 Minuten lang, vorzugsweise 5 bis 30 Minuten lang, auf die Keratinfaser einwirken, spült sodann die Keratinfaser mit Wasser aus und trocknet sie. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird die Keratinfaser getrocknet.

Außerdem ist es möglich, bei Färbungen von unterschiedlich stark geschädigtem Haar (beispielsweise Nachfärbungen schon oxidativ gefärbter Haarpartien), auf die vorgeschädigten Haarpartien (zum Beispiel die Haarspitzen) die Farbträgermasse ohne Oxidationsmittel -pur oder nur mit einer weiteren sauren, neutralen oder basischen wässrigen Komponente verdünnt- aufzutragen, während man auf die gering oder gar nicht vorgeschädigten Haarpartien (beispielsweise den Haaransatz und die Haarlängen) die mit dem Oxidationsmittel vermischte Farbträgermasse aufträgt. Die zur Verdünnung eingesetzte wässrige Komponente kann die vorstehend genannten üblichen Zusätze für Lösungen, Cremes, Emulsionen oder Gelen enthalten. Dieses Verfahren ermöglicht auf die Haarbeschaffenheit abgestimmte Färbungen, die sich durch einen haarschonenden Ausgleich zwischen Ansatz und Spitzen auszeichnen, was bei der Verwend ung von üblichen oxidativen Haarfärbemitteln nicht möglich ist, da zum Kuppeln der Farbstoffvorstufen immer ein Oxidationsmittel benötigt wird.

Das erfindungsgemäße Färbemittel ermöglicht Färbungen, die sich durch ihre besondere Farbintensität und Leuchtkraft, einen guten Farbausgleich zwischen geschädigtem und ungeschädigtem Haar (zum Beispiel zwischen Haarspitzen und Haarnachwuchs) sowie durch eine besonders gute Lichtechtheit und Schweißbeständigkeit auszeichnen.

Die Farbstoffe sind zum Teil an sich bekannt. Die Herstellung der Farbstoffe der Formel (I) kann in Analogie zur bekannten Herstellungsverfahren, wie zum Beispiel via Azokupplung von 2-Aminothiazolderivaten mit N-Benzyl-Aminophenyl-Derivaten, und nachfolgende Quatemisierung, oder via oxidativen
Kondensation von Thiazolon-hydrazonen mit N-Benzylaminophenyl-Derivaten erfolgen.

Die nachfolgenden Beispiele sollen den Gegenstand der Erfindung näher erläutern, ohne diesen hierauf zu beschränken.

### Beispiele

### Färbebeispiele 1 bis 8: Vergleichsversuche

| | |
|---|---|
| 0,53 g | Farbstoff der Formel (I) |
| 5,00 g | Ethanol |
| 4,00 g | Decylglucosid |
| 0,20 g | Ethylendiaminotetraessigsäure-Dinatriumsalz |
| ad 100,00 g | Wasser, vollentsalzt |

### A) Erfindungsgemäß

5 g der vorstehenden Färbelösung werden mit 5 g einer 9 %igen Wasserstoffperoxidlösung vermischt. Die Färbelösung wird durch Zugabe von Ammoniak auf den gewünschten pH-Wert eingestellt. Das erhaltene gebrauchsfertige Haarfärbemittel wird auf zu 80% ergrautes Haar aufgetragen und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit lauwarmem Wasser ausgespült und sodann getrocknet.

### B) Nicht-erfindunqsgemäß (ohne Wasserstoffperoxid)

5 g der vorstehenden Färbelösung werden mit 5 g Wasser vermischt. Die Färbelösung wird durch Zugabe von Ammoniak auf den gewünschten pH-Wert eingestellt. Das erhaltene gebrauchsfertige Haarfärbemittel wird auf zu 80% ergrautes Haar aufgetragen und mit einem Pinsel gleichmäßig verteilt wird. Nach einer Einwirkungszeit von 30 Minuten bei 40 °C wird das Haar mit lauwarmem Wasser ausgespült, mit einem Shampoo gewaschen, mit lauwarmem Wasser gespült und sodann getrocknet.

Die Färbeergebnisse sind in der nachfolgenden Tabelle 1 zusammengefasst.

**Tabelle 1**

| **Bsp.** | **Verbindung der Formel (I)** | **pH Wert der Mischung** | **Farbton nach dem Färben** |
|---|---|---|---|
| | | | **A: mit H2O2** |
| | | | **B: ohne H2O2** |
| **1.** | 3-Methyl-2-[[4-[methyl-(phenylmethyl)amino]-phenyl]azo]-thiazolium-monomethylsulfat | 9,5 | A) helles Königsblau |
| | | | B) Königsblau |
| **2.** | 3,4-Dimethyl-2-[[4-[methyl-(phenylmethyl)amino]-phenyl]azo]-thiazolium-monomethylsulfat | 9,3 | A) helles Königsblau |
| | | | B) Königsblau |
| **3.** | 3,5-Dimethyl-2-[[4-[methyl-(phenylmethyl)amino]-phenyl]azo]-thiazolium-monomethylsulfat | 9,3 | A) helles Königsblau |
| | | | B) Königsblau |
| **4.** | 3,4,5-Trimethyl-2-[[4-[methyl-(phenylmethyl)-amino]phenyl]azo]-thiazolium-monomethylsulfat | 9,1 | A) helles Königsblau |
| | | | B) Königsblau |
| **5.** | 2-[[4-[Ethyl(phenylmethyl)-amino]2-methylphenyl]azo]-3-methyl-thiazolium-monomethylsulfat | 9,4 | A) helles Königsblau |
| | | | B) Königsblau |
| **6.** | 2-[[4-[Ethyl(phenylmethyl)-amino]2-methylphenyl]azo]-3,5-dimethyl-thiazolium-monomethylsulfat | 9,4 | A) helles Königsblau |
| | | | B) Königsblau |
| **7.** | 2-[[4-[Ethyl(phenylmethyl)-amino]2-methylphenyl]azo]-3,4-dimethyl-thiazolium-monomethylsulfat | 9,4 | A) helles Königsblau |
| | | | B) Königsblau |
| **8.** | 2-[[4-[Ethyl(phenylmethyl)-amino]2-methylphenyl]azo]-3,4,5-trimethyl-thiazolium-monomethylsulfat | 9,4 | A) helles Königsblau |
| | | | B) Königsblau |

### Beispiele 9 bis 13: stark aufhellendes Färbemittel

### Aufhellende Pulverbasis

| | |
|---|---|
| 20,0 g | Kaliumpersulfat |
| 30,0 g | Ammoniumpersulfat |
| 24,0 g | Natriumsilicat |
| 12,5 g | Magnesiumoxid |
| 5,0 g | Hydroxycellulose |
| 6,0 g | Seifenperlen |
| 2,0 g | disperse Kieselsäure |
| 0,5 g | Dinatrium-EDTA |

0,1 g Farbstoff der Formel (I) gemäß Tabelle 2 werden in 9,9 g der aufhellenden Pulverbasis eingearbeitet. Anschließend wird das so erhaltene Färbepulver mit 20 g einer 12%igen wässrigen Peroxidlösung vermischt und in einer Mischschale homogen verrührt, und sodann auf trockene dunkelblonde Naturhaare aufgebracht. Nach einer Einwirkzeit von 30 Minuten bei 40°C werden die Haare mit lauwarmen Wasser ausgespühlt, mit einem Shampoo gewaschen, mit lauwarmen Wasser ausgespühlt und sodann getrocknet.

Die verwendeten Farbstoffe sowie die Färbeergebnisse sind in der nachfolgenden Tabelle 2 zusammengefasst

**Tabelle 2**

| **Bsp.** | **Verbindung der Formel (I)** | **Farbton nach dem Färben** |
|---|---|---|
| **9.** | 3-Methyl-2-[[4-[methyl-(phenyl-methyl)amino]-phenyl]azo]-thiazolium-monomethylsulfat | Stahlblau |
| **10.** | 3,5-Dimethyl-2-[[4-[methyl-(phenyl-methyl)amino]-phenyl]-azo]-thiazolium-monomethylsulfat | Stahlblau |
| **11.** | 3,4,5-Trimethyl-2-[[4-[methyl-(phenylmethyl)-amino]phenyl]-azo]-thiazolium-monomethylsulfat | Stahlblau |
| **12.** | 2-[[4-[Ethyl(phenylmethyl)-amino]2-methylphenyl]azo]-3-methyl-thiazolium-monomethylsulfat | Stahlblau |
| **13.** | 2-[[4-[Ethyl(phenylmethyl)-amino]2-methylphenyl]azo]-3,5-dimethyl-thiazolium-monomethylsulfat | Stahlblau |

Alle Prozentangaben in der vorliegenden Anmeldung stellen, sofern nicht anders angegeben, Gewichtsprozente dar.

## Patentansprüche

1. Mittel zur gleichzeitigen Aufhellung und Färbung von Keratinfasern, **dadurch gekennzeichnet, dass** es (a) ein Oxidationsmittel sowie (b) mindestens einen Thiazoliumazofarbstoff der Formel (I) enthält, und (c) einen basischen pH-Wert aufweist, 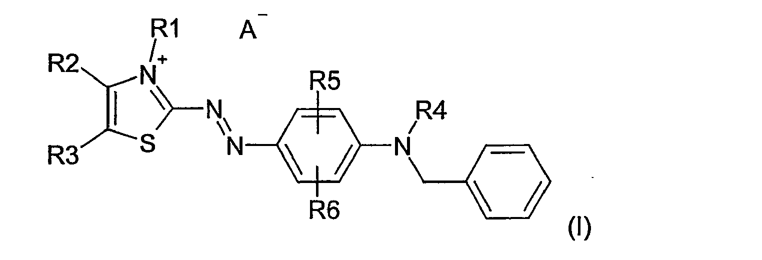 wobei
**R1** eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₆)Alkoxy-(C₁-C₁₂)-alkylgruppe, eine Amino-(C₁-C₁₂)-alkylgruppe, eine (C₁-C₆)-Alkylamino-(C₁-C₁₂)-alkylgruppe, eine Di-(C₁-C₆)-alkylamino-(C₁-C₁₂)-alkylgruppe, eine Cyano-(C₁-C₁₂)-alkylgruppe, eine substituierte oder unsubstituierte Phenylgruppe oder eine substituierte oder unsubstituierte Benzylgruppe darstellt;
**R2** und **R3** gleich oder verschieden sein können und unabhängig voneinander Wasserstoff, ein Halogenatom, eine gesättigte oder ungesättigte (C₁-C₁₂)-Alkylgruppe, eine (C₁-C₁₂)-Alkoxygruppe, eine Nitrogruppe, eine Aminogruppe, eine (C₁-C₁₂)-Alkylaminogruppe, eine Di(C₁-C₁₂)-alkylaminogruppe, eine (C₁-C₁₂)-Hydroxyalkylaminogruppe, eine Di(C₁-C₁₂)-hydroxyalkylaminogruppe, eine substituierte oder unsubstituierte Phenylgruppe, oder eine substituierte oder unsubstituierte Heteroarylgruppe darstellen;
**R4** gleich Wasserstoff, einer gesättigten oder ungesättigten (C₁-C₁₂)-Alkylgruppe, einer mit einem Halogenatom substituierten (C₁-C₁₂)-Alkylgruppe, einer Hydroxy-(C₁-C₁₂)-alkylgruppe, einer Amino-(C₁-C₁₂)-alkylgruppe oder einer Benzylgruppe ist;
**R5** und **R6** gleich oder verschieden sein können und unabhängig voneinander Wasserstoff, ein Halogenatom, eine gesättigte oder ungesättigte (C₁-C₁₂)-Alkylgruppe, eine Hydroxygruppe, eine (C₁-C₁₂)-Alkoxygruppe, eine Cyanogruppe, eine Nitrogruppe, eine Aminogruppe, eine (C₁-C₁₂)-Alkylaminogruppe, oder eine Di(C₁-C₁₂)-alkylaminogruppe darstellen; und
**A⁻** gleich einem Anion einer organischen oder anorganischen Säure ist.

2. Mittel nach Anspruch 1, **dadurch gekennzeichnet, dass R1** gleich einer gesättigten oder ungesättigten (C₁-C₁₂)-Alkylgruppe ist.

3. Mittel nach Anspruch 2, **dadurch gekennzeichnet, dass R4** gleich einer gesättigten oder ungesättigten (C₁-C₁₂)-Alkylgruppe ist.

4. Mittel nach einem der Ansprüche 1 bis 3, **dadurch gekennzeichnet, dass A⁻** gleich einem Chlorid-, Bromid-, Jodid-, Hydrogensulfat-, Sulfat-, Toluolsulfonat-, Benzolsulfonat-, Monomethylsulfat-, Hexafluorphosphat-, Hexafluorantimonat-, Tetrafluorborat-, Tetraphenylborat-, Formiat-, Acetat-oder Propionat-Anion ist.

5. Mittel nach einem der Ansprüche 1 bis 4, **dadurch gekennzeichnet, dass** die Verbindung der Formel (I) ausgewählt ist aus 3-Methyl-2-[[4-[methyl(phenylmethyl)amino]phenyl]azo]-thiazolium-chlorid, 3-Methyl-2-[[4-[methyl(phenylmethyl)amino]phenyl]azo]-thiazolium-bromid, 3-Methyl-2-[[4-[methyl(phenylmethyl)amino]phenyl]-azo]-thiazolium-monomethylsulfat, 3-Methyl-2-[[4-[methyl(phenylmethyl)amino]phenyl]-azo]-thiazolium-acetat, 3,4-Dimethyl-2-[[4-[methyl(phenylmethyl)amino]phenyl]azo]-thiazolium-chlorid, 3,4-Dimethyl-2-[[4-[methyl(phenylmethyl)amino]phenyl]azö]-thiazolium-bromid, 3,4-Dimethyl-2-[[4-[methyl(phenylmethyl)amino]phenyl]azo]-thiazolium-monomethylsulfat, 3,4-Dimethyl-2-[[4-[methyl(phenylmethyl)-amino]phenyl]azo]-thiazolium-acetat, 3,5-Dimethyl-2-[[4-[methyl(phenylmethyl)amino]-phenyl]azo]-thiazolium-chlorid, 3,5-Dimethyl-2-[[4-[methyl(phenylmethyl)-amino]-phenyl]azo]-thiazolium-bromid, 3,5-Dimethyl-2-[[4-[methyl(phenylmethyl)-amino]phenyl]azo]-thiazolium-monomethylsulfat, 3,5-Dimethyl-2-[[4-[methyl(phenylmethyl)amino]phenyl]azo]-thiazolium-acetat, 3,4,5-Trimethyl-2-[[4-[methyl-(phenylmethyl)-amino]phenyl]azo]-thiazolium-chlorid, 3,4,5-Trimethyl-2-[[4-[methyl-(phenylmethyl)amino]-phenyl]azo]-thiazolium-bromid, 3,4,5-Trimethyl-2-[[4-[methyl(phenylmethyl)amino]phenyl]azo]-thiazolium-monomethylsulfat, 3,4,5-Trimethyl-2-[[4-[methyl(phenylmethyl)amino]phenyl]azo]-thiazolium-acetat, 2-[[4-[Ethyl(phenylmethyl)amino]2-methyl-phenyl]azo]-3-methylthiazolium-chlorid, 2-[[4-[Ethyl(phenylmethyl)amino]-2-methylphenyl]azo]-3-methyl-thiazolium-bromid, 2-[[4-[Ethyl(phenyl-methyl)amino]2-methylphenyl]azo]-3-methyl-thiazolium-monomethylsulfat, 2-[[4-[Ethyl(phenyl-methyl)amino]2-methylphenyl]azo]-3-methylthiazolium-acetat, 2-[[4-[Ethyl(phenylmethyl)-amino]2-methylphenyl]azo]-3,4-dimethyl-thiazolium-chlorid, 2-[[4-[Ethyl(phenylmethyl)-amino]2-methylphenyl]azo]-3,4-dimethyl-thiazolium-bromid, 2-[[4-[Ethyl(phenylmethyl)-amino]2-methylphenyl]azo]-3,4-dimethyl-thiazoliummonomethylsulfat, 2-[[4-[Ethyl(phenylmethyl)-amino]2-methylphenyl]azo]-3,4-dimethyl-thiazolium-acetat, 2-[[4-[Ethyl(phenylmethyl)-amino]2-methylphenyl]azo]-3,5-dimethyl-thiazolium-chlorid, 2-[[4-[Ethyl(phenylmethyl)-amino]2-methylphenyl]azo]-3,5-dimethyl-thiazolium-bromid, 2-[[4-[Ethyl(phenylmethyl)-amino]2-methylphenyl]azo]-3,5-dimethylthiazolium-monomethylsulfat, 2-[[4-[Ethyl(phenylmethyl)-amino]2-methylphenyl]azo]-3,5-dimethyl-thiazolium-acetat, 2-[[4-[Ethyl(phenylmethyl)amino]2-methylphenyl]azo]-3,4,5-trimethyl-thiazolium-chlorid, 2-[[4-[Ethyl(phenylmethyl)amino]2-methylphenyl]azo]-3,4,5-trimethyl-thiazolium-bromid und 2-[[4-[Ethyl(phenylmethyl)amino]2-methylphenyl]-azo]-3,4,5-trimethyl-thiazolium-monorriethylsulfat und 2-[[4-[Ethyl-(phenylmethyl)amino]2-methylphenyl]azo]-3,4,5-trimethyl-thiazolium-acetat.

6. Mittel nach einem der Ansprüche 1 bis 5, **dadurch gekennzeichnet, dass** die Verbindung der Formel (I) in einer Menge von 0,01 bis 10 Gewichtsprozent enthalten ist.

7. Mittel nach einem der Ansprüche 1 bis 6, **dadurch gekennzeichnet, dass** es zusätzlich mindestens einen weiteren oxidationsstabilen direktziehenden Farbstoff und/oder Oxidationsfarbstoffvorstufe enthält.

8. Mittel nach Anspruch 1 bis 7, **dadurch gekennzeichnet, dass** das gebrauchsfertige Färbemittel einen pH-Wert von 8 bis 11 aufweist.

9. Mittel nach einem der Ansprüche 1 bis 8, **dadurch gekennzeichnet, dass** das Oxidationsmittel ausgewählt ist aus Wasserstoffperoxid oder dessen Additionsverbindungen an Harnstoff, Melamin, Natriumborat oder Natriumcarbonat.

10. Mittel nach einem der Ansprüche 1 bis 9, **dadurch gekennzeichnet, dass** es zusätzlich ein Persulfat enthält.

11. Mittel nach einem der Ansprüche 1 bis 10, **dadurch gekennzeichnet, dass** es in Form eines 2-Komponenten-Mittels, bestehend aus einer mindestens einen Thiazoliumazofarbstoff der Formel (I) enthaltenden Farbträgermasse (A) und einer ein Oxidationsmittel enthaltenden Komponente (B), vorliegt.

12. Mittel nach einem der Ansprüche 1 bis 11, **dadurch gekennzeichnet, dass** es ein Haarfärbemittel ist.

## Claims

1. Agent for the simultaneous lightening and dyeing of keratin fibres, **characterized in that** it comprises (a) an oxidizing agent, and (b) at least one thiazolium azo dye of the formula (I), and (c) has a basic pH, 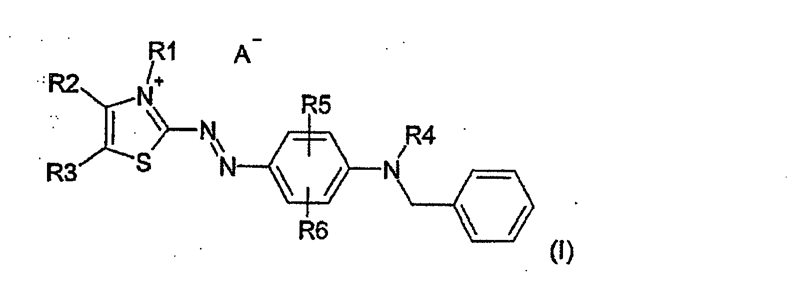 where
**R1** is a saturated or unsaturated (C₁-C₁₂)-alkyl group, a (C₁-C₁₂)-alkyl group substituted by a halogen atom, a hydroxyl- (C₁-C₁₂) -alkyl group, a (C₁-C₆) -alkoxy- (C₁-C₁₂) - alkyl group, an amino-(C₁-C₁₂) -alkyl group, a (C₁-C₆) - alkylamino- (C₁-C₁₂) -alkyl group, a di (C₁-C₆) -alkylamino-(C₁-C₁₂)-alkyl group, a cyano-(C₁-C₁₂)-alkyl group, a substituted or unsubstituted phenyl group or a substituted or unsubstituted benzyl group;
R2 and R3 may be identical or different and, independently of one another, are hydrogen, a halogen atom, a saturated or unsaturated (C₁-C₁₂)-alkyl group, a (C₁-C₁₂)-alkoxy group, a nitro group, an amino group, a (C₁-C₁₂)-alkylamino group, a di (C₁-C₁₂)-alkylamino group, a (C₁-C₁₂)-hydroxyalkylamino group, a di (C₁-C₁₂) - hydroxyalkylamino group, a substituted or unsubstituted phenyl group, or a substituted or unsubstituted heteroaryl group;
**R4** is hydrogen, a saturated or unsaturated (C₁-C₁₂)-alkyl group, a (C₁-C₁₂)-alkyl group substituted by a halogen atom, a hydroxyl-(C₁-C₁₂)-alkyl group, an amino-(C₁-C₁₂)-alkyl group or a benzyl group;
**R5** and **R6** may be identical or different and, independently of one another, are hydrogen, a halogen atom, a saturated or unsaturated (C₁-C₁₂)-alkyl group, a hydroxyl group, a (C₁-C₁₂)-alkoxy group, a cyano group, a nitro group, an amino group, a (C₁-C₁₂)-alkylamino group, or a di(C₁-C₁₂)-alkylamino group; and
**A⁻** is an anion of an organic or inorganic acid.

2. Agent according to Claim 1, **characterized in that R1** is a saturated or unsaturated (C₁-C₁₂)-alkyl group.

3. Agent according to Claim 2, **characterized in that R4** is a saturated or unsaturated (C₁-C₁₂)-alkyl group.

4. Agent according to one of Claims 1 to 3, **characterized in that A⁻** is a chloride, bromide, iodide, hydrogensulphate, sulphate, toluenesulphonate, benzene-sulphonate, monomethylsulphate, hexafluorophosphate, hexafluoroantimonate, tetrafluoroborate, tetraphenylborate, formate, acetate or propionate anion.

5. Agent according to one of Claims 1 to 4, **characterized in that** the compound of the formula (I) is chosen from 3-methyl-2-[[4-[methyl(phenylmethyl)-amino]phenyl]azo]thiazolium chloride, 3-methyl-2-[[4-[methyl(phenylmethyl)amino]phenyl]azo]thiazolium bromide, 3-methyl-2-[[4-[methyl(phenylmethyl)amino]-phenyl]azo]thiazolium monomethylsulphate, 3-methyl-2-[[4-[methyl(phenylmethyl)amino]phenyl]azo]thiazolium acetate, 3,4-dimethyl-2-[[4-[methyl(phenylmethyl)-amino]phenyl]azo]thiazolium chloride, 3,4-dimethyl-2-[[4-[methyl(phenylmethyl)amino]phenyl]azo]thiazolium bromide, 3,4-dimethyl-2-[[4-[methyl(phenylmethyl)-amino]phenyl]azo]thiazolium monomethylsulphate, 3,4-dimethyl-2-[[4-[methyl(phenylmethyl)amino]phenyl]azo]-thiazolium acetate, 3,5-dimethyl-2-[[4-[methyl(phenylmethyl)amino]phenyl]azo]thiazolium chloride, 3,5-dimethyl-2-[[4-[methyl(phenylmethyl)-amino]phenyl]azo]thiazolium bromide, 3,5-dimethyl-2-[[4-[methyl(phenylmethyl)amino]phenyl]azo]thiazolium monomethylsulphate, 3,5-dimethyl-2-[[4-[methyl(phenylmethyl)amino]phenyl]azo]thiazolium acetate, 3,4,5-trimethyl-2-[[4-[methyl(phenylmethyl)amino]phenyl]azo]-thiazolium chloride, 3,4,5-trimethyl-2-[[4-[methyl(phenylmethyl)amino]phenyl]azo]thiazolium bromide, 3,4,5-trimethyl-2-[[4-[methyl(phenylmethyl)-amino]phenyl]azo]thiazolium monomethylsulphate, 3,4,5-trimethyl-2-[[4-[methyl(phenylmethyl)amino]phenyl]azo]-thiazolium acetate, 2-[[4-[ethyl(phenylmethyl)amino]-2-methylphenyl]azo]-3-methylthiazolium chloride, 2-[[4-[ethyl(phenylmethyl)amino]-2-methylphenyl]azo]-3-methylthiazolium bromide, 2-[[4-[ethyl(phenylmethyl)-amino]-2-methylphenyl]azo]-3-methylthiazolium monomethylsulphate, 2-[[4-[ethyl(phenylmethyl)amino]-2-methylphenyl]azo]-3-methylthiazolium acetate, 2-[[4-[ethyl(phenylmethyl)amino]-2-methylphenyl]azo]-3,4-dimethylthiazolium chloride, 2-[[4-[ethyl(phenylmethyl)amino]-2-methylphenyl]azo]-3,4-dimethyl-thiazolium bromide, 2-[[4-[ethyl(phenylmethyl)amino]-2-methylphenyl]azo]-3,4-dimethylthiazolium monomethylsulphate, 2-[[4-[ethyl(phenylmethyl)amino]-2-methylphenyl]azo]-3,4-dimethylthiazolium acetate, 2-[[4-[ethyl(phenylmethyl)amino]-2-methylphenyl]azo]-3,5-dimethylthiazolium chloride, 2-[[4-[ethyl(phenylmethyl)amino]-2-methylphenyl]azo]-3,5-dimethyl-thiazolium bromide, 2-[[4-[ethyl(phenylmethyl)amino]-2-methylphenyl]azo]-3,5-dimethylthiazolium monomethylsulphate, 2-[[4-[ethyl(phenylmethyl)amino]-2-methylphenyl]azo]-3,5-dimethylthiazolium acetate, 2-[[4-[ethyl(phenylmethyl)amino]-2-methylphenyl]azo]-3,4,5-trimethylthiazolium chloride, 2-[[4-[ethyl(phenylmethyl)amino]-2-methylphenyl]azo]-3,4,5-trimethyl-thiazolium bromide and 2-[[4-[ethyl(phenylmethyl)-amino]-2-methylphenyl]azo]-3,4,5-trimethylthiazolium monomethylsulphate and 2-[[4-[ethyl(phenylmethyl)-amino]-2-methylphenyl]azo]-3,4,5-trimethylthiazolium acetate.

6. Agent according to one of Claims 1 to 5, **characterized in that** the compound of the formula (I) is present in an amount of from 0.01 to 10% by weight.

7. Agent according to one of Claims 1 to 6, **characterized in that** it additionally comprises at least one further oxidation-stable direct dye and/or oxidation dye precursor.

8. Agent according to Claim 1 to 7, **characterized in that** the ready-to-use dye has a pH of from 8 to 11.

9. Agent according to one of Claims 1 to 8, **characterized in that** the oxidizing agent is chosen from hydrogen peroxide or its addition compounds onto urea, melamine, sodium borate or sodium carbonate.

10. Agent according to one of Claims 1 to 9, **characterized in that** it additionally comprises a persulphate.

11. Agent according to one of Claims 1 to 10, **characterized in that** it is in the form of a 2-component agent consisting of a colour carrier mass (A) comprising at least one thiazolium azo dye of the formula (I), and a component (B) comprising an oxidizing agent.

12. Agent according to one of Claims 1 to 11, **characterized in that** it is a hair dye.

## Revendications

1. Composition destinée à éclaircir et colorer simultanément des fibres de kératine, **caractérisée en ce que** (a) elle contient un oxydant ainsi que (b) au moins un colorant azoïque thiazolium de formule (I), et (c) présente un pH basique 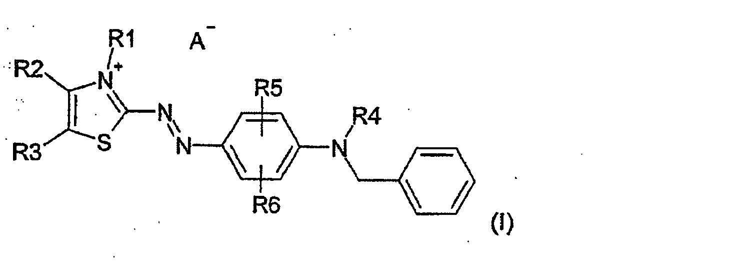 formule dans laquelle
**R1** représente un groupe alkyle en C₁-C₁₂ saturé ou insaturé, un groupe alkyle en C₁-C₁₂ substitué par un atome d'halogène, un groupe hydroxyalkyle en C₁-C₁₂, un groupe alcoxy(C₁-C₆) -alkyle (C₁-C₁₂), un groupe aminoalkyle en C₁-C₁₂, un groupe alkyl (C₁-C₆) amino-alkyle (C₁-C₁₂), un groupe dialkyl (C₁-C₆) amino-alkyle (C₁-C₁₂), un groupe cyanoalkyle(C₁-C₁₂), un groupe phényle substitué ou non substitué ou un groupe benzyle substitué ou non substitué ;
R2 et R3 peuvent être identiques ou différents et représentent, indépendamment l'un de l'autre, un atome d'hydrogène, un atome d'halogène, un groupe alkyle en C₁-C₁₂ saturé ou insaturé, un groupe alcoxy en C₁-C₁₂, un groupe nitro, un groupe amino, un groupe alkyl(C₁-C₁₂)amino, un groupe dialkyl(C₁-C₁₂)amino, un groupe hydroxyalkyl(C₁-C₁₂) amino, un groupe dihydroxyalkyl(C₁-C₁₂)amino, un groupe phényle substitué ou non substitué ou un groupe hétéroaryle substitué ou non substitué ;
**R4** représente un atome d'hydrogène, un groupe alkyle en C₁-C₁₂ saturé ou insaturé, un groupe alkyle en C₁-C₁₂ substitué par un atome d'halogène, un groupe hydroxyalkyle en C₁-C₁₂, un groupe aminoalkyle en C₁-C₁₂ ou un groupe benzyle ;
**R5** et **R6** peuvent être identiques ou différents et représentent, indépendamment l'un de l'autre, un atome d'hydrogène, un atome d'halogène, un groupe alkyle en C₁-C₁₂ saturé ou insaturé, un groupe hydroxy, un groupe alcoxy en C₁-C₁₂, un groupe cyano, un groupe nitro, un groupe amino, un groupe alkyl (C₁-C₁₂) amino ou un groupe dialkyl (C₁-C₁₂)-amino ; et
**A⁻** est un anion d'un acide organique ou minéral.

2. Composition selon la revendication 1, **caractérisée en ce que R1** est un groupe alkyle en C₁-C₁₂ saturé ou insaturé.

3. Composition selon la revendication 2, **caractérisée en ce que R4** est un groupe alkyle en C₁-C₁₂ saturé ou insaturé.

4. Composition selon l'une quelconque des revendications 1 à 3, **caractérisée en ce que** A⁻ est un anion chlorure, bromure, iodure, hydrogénosulfate, sulfate, toluènesulfonate, benzènesulfonate, monométhylsulfate, hexafluorophosphate, hexafluoroantimonate, tétrafluoroborate, tétraphénylborate, formiate, acétate ou propionate.

5. Composition selon l'une quelconque des revendications 1 à 4, **caractérisée en ce que** le composé de formule (I) est choisi parmi le chlorure de 3-méthyl-2-[[4-[méthyl(phénylméthyl)-amino]phényl]azo]-thiazolium, le bromure de 3-méthyl-2-[[4-[méthyl(phénylméthyl)amino]phényl]-azo]-thiazolium, le monométhylsulfate de 3-méthyl-2-[[4-[méthyl(phénylméthyl)amino]phényl]azo]-thiazolium, l'acétate de 3-méthyl-2-[[4-[méthyl(phénylméthyl)amino]phényl]azo]-thiazolium, le chlorure de 3,4-diméthyl-2-[[4-[méthyl(phénylméthyl)amino]phényl]azo]-thiazolium, le bromure de 3,4-diméthyl-2-[[4-[méthyl(phénylméthyl)amino]-phényl]azo]-thiazolium, le monométhylsulfate de 3,4-diméthyl-2-[[4-[méthyl(phénylméthyl)amino]-phényl]azo]-thiazolium, l'acétate de 3,4-diméthyl-2-[[4-[méthyl(phénylméthyl)amino]phényl]azo]-thiazolium, le chlorure de 3,5-diméthyl-2-[[4-[méthyl(phénylméthyl)amino]phényl]azo]-thiazolium, le bromure de 3,5-diméthyl-2-[[4-[méthyl(phénylméthyl)amino]phényl]azo]-thiazolium, le monométhylsulfate de 3,5-diméthyl-2-[[4-[méthyl-(phénylméthyl)amino]phényl]azo]-thiazolium, l'acétate de 3,5-diméthyl-2-[[4-[méthyl(phénylméthyl)amino]phényl]azo]-thiazolium, le chlorure de 3,4,5-triméthyl-2-[[4-[méthyl(phénylméthyl)-amino]phényl]azo]-thiazolium, le bromure de 3,4,5-triméthyl-2-[[4-[méthyl(phénylméthyl)amino]-phényl]azo]-thiazolium, le monométhylsulfate de 3,4,5-triméthyl-2-[[4-[méthyl(phénylméthyl)amino]-phényl]azo]-thiazolium, l'acétate de 3,4,5-triméthyl-2-[[4-[méthyl(phénylméthyl)amino]-phényl]azo]-thiazolium, le chlorure de 2-[[4-[éthyl(phénylméthyl)amino]-2-méthylphényl]azo]-3-méthylthiazolium, le bromure de 2-[[4-[éthyl(phénylméthyl)amino]-2-méthylphényl]azo]-3-méthylthiazolium, le monométhylsulfate de 2-[[4-[éthyl(phénylméthyl)amino]-2-méthylphényl]azo]-3-méthylthiazolium, l'acétate de 2-[[4-[éthyl-(phénylméthyl)amino]-2-méthylphényl]azo]-3-méthylthiazolium, le chlorure de 2-[[4-[éthyl(phénylméthyl)amino]-2-méthylphényl]azo]-3,4-diméthylthiazolium, le bromure de 2-[[4-[éthyl(phénylméthyl)amino]-2-méthylphényl]azo]-3,4-diméthylthiazolium, le monométhylsulfate de 2-[[4-[éthyl(phénylméthyl)amino]-2-méthylphényl]-azo]-3,4-diméthylthiazolium, l'acétate de 2-[[4-[éthyl(phénylméthyl)amino]-2-méthylphényl]azo]-3,4-diméthylthiazolium, le chlorure de 2-[[4-[éthyl(phénylméthyl)amino]-2-méthylphényl]azo]-3,5-diméthylthiazolium, le bromure de 2-[[4-[éthyl(phénylméthyl)amino]-2-méthylphényl]azo]-3,5-diméthylthiazolium, le monométhylsulfate de 2-[[4-[éthyl(phénylméthyl)amino]-2-méthylphényl]-azo]-3,5-diméthylthiazolium, l'acétate de 2-[[4-[éthyl(phénylméthyl)amino]-2-méthylphényl]azo]-3,5-diméthylthiazolium, le chlorure de 2-[[4-[éthyl(phénylméthyl)amino]-2-méthylphényl]azo]-3,4,5-triméthylthiazolium, le bromure de 2-[[4-[éthyl(phénylméthyl)amino]-2-méthylphényl]azo]-3,4,5-triméthylthiazolium, le monométhylsulfate de 2-[[4-[éthyl(phénylméthyl)amino]-2-méthylphényl]-azo]-3,4,5-triméthylthiazolium, l'acétate de 2-[[4-[éthyl(phénylméthyl)amino]-2-méthylphényl]-azo]-3,4,5-triméthylthiazolium.

6. Composition selon l'une quelconque des revendications 1 à 5, **caractérisée en ce que** le composé de formule (I) est contenu en une quantité de 0,01 à 10 % en poids.

7. Composition selon l'une quelconque des revendications 1 à 6, **caractérisée en ce qu'**elle contient en outre au moins un autre colorant direct stable vis-à-vis de l'oxydation et/ou des précurseurs de colorants d'oxydation.

8. Composition selon l'une quelconque des revendications 1 à 7, **caractérisée en ce que** la composition de teinture prête à l'emploi présente un pH de 8 à 11.

9. Composition selon l'une quelconque des revendications 1 à 8, **caractérisée en ce que** l'oxydant est choisi parmi le peroxyde d'hydrogène ou ses composés d'addition par fixation sur l'urée, la mélamine, le borate de sodium ou le carbonate de sodium.

10. Composition selon l'une quelconque des revendications 1 à 9, **caractérisée en ce qu'**elle contient en outre un persulfate.

11. Composition selon l'une quelconque des revendications 1 à 10, **caractérisée en ce qu'**elle se trouve sous forme d'une composition à deux composants, constituée d'une matière colorante (A) contenant au moins un colorant azoïque thiazolium de formule (I) et d'un composant (B) contenant un oxydant.

12. Composition selon l'une quelconque des revendications 1 à 11, **caractérisée en ce qu'**il s'agit d'une composition de teinture pour cheveux.
